# DEMANDE DE BREVET EUROPEEN

(11) **EP 2 898 851 A1**
(43) Date de publication de la demande: **29.07.2015**
(21) Numéro de dépôt: 14152944.6
(22) Date de dépôt: 28.01.2014
(51) Int. Cl.: A61C 7/36, A61F 5/56

(54) **Appareil dentaire pour le réglage du positionnement de la mâchoire inférieure d'un patient par rapport à sa mâchoire supérieure**

(71) Demandeur: Valceschini, Michel, 1808 Les Monts-de-Corsier (CH)
(72) Inventeur: Valceschini, Michel, 1808 Les Monts-de-Corsier (CH)
(74) Mandataire: Ganguillet, Cyril

(57) **Abrégé**

L'invention est relative à un appareil dentaire (1) pour le réglage du positionnement de la mâchoire inférieure d'un patient par rapport à sa mâchoire supérieure, ledit appareil comprenant une gouttière supérieure (2) et une gouttière inférieure (3) destinées à chausser, respectivement, les dents de la mâchoire supérieure et de la mâchoire inférieure, dans lequel lesdites gouttières (2, 3) sont reliées entre elles au moyen d'un fil ressort (4) possédant une forme sensiblement en U, ledit fil ressort (4) étant disposé en position occlusale entre lesdites gouttières (2, 3) sans dépasser dans les zones vestibulaires de la bouche du patient, dans lequel chacune des extrémités (4c) du fil ressort (4) est montée de manière pivotante sur une première desdites gouttières inférieure (3) ou supérieure (2) autour d'un axe de pivotement (X1X1' ; X2X2') parallèle au plan occlusal (Po) de contact des dents des mâchoires inférieure et supérieure, dans lequel le fil ressort (4) est relié de manière pivotante et amovible à une seconde desdites gouttières inférieure (3) ou supérieure (2) au moyen d'un crochet (6) fixé de manière amovible ou non amovible sur ladite seconde gouttière, ledit crochet (6) étant positionné de telle sorte qu'il produise un décalage (d) entre les première et seconde gouttières (2, 3) dans au moins une direction perpendiculaire à l'axe de pivotement (X3X3') du fil ressort (4) par rapport à la seconde gouttière.

## Description

### Domaine technique

La présente invention a pour objet un appareil dentaire pour le réglage du positionnement de la mâchoire inférieure d'un patient par rapport à sa mâchoire supérieure.

### Etat de la technique

Les appareils dentaires pour le réglage du positionnement de la mâchoire inférieure sont utilisés d'une part en orthodontie et d'autre part pour prévenir le ronflement et les apnées obstructives du sommeil.

On sait notamment que les manifestations pathologiques du sommeil telles que ronflement ou apnée du sommeil peuvent être prévenues en maintenant la mâchoire inférieure dans une position avancée par rapport à la mâchoire supérieure. De nombreux appareils capables de maintenir les mâchoires dans cet état ont déjà été proposés, mais ils présentent pour la plupart des défauts qui entravent leur application généralisée.

Ainsi, on connaît par la publication US 2011/0277774 un appareil oral destiné à prévenir le ronflement et comprenant deux parties avec chacune une gouttière dentaire adaptée à l'un des maxillaires, supérieur et inférieur respectivement, lesdites gouttières étant reliées entre elles au moyen d'un fil métallique destiné à être partiellement reçu à l'intérieur d'organes de fixation correspondants desdites gouttières. Dans cet appareil connu, un des organes de fixation est un tube creux supérieur qui est noyé dans la gouttière dentaire recouvrant le maxillaire supérieur et dans lequel le fil métallique est partiellement logé, ledit tube supérieur étant positionné à l'avant de la bouche. Les deux autres organes de fixation consistent en deux tubes creux inférieurs qui sont partiellement noyés dans la gouttière dentaire recouvrant le maxillaire inférieur et dans lesquels les extrémités du fil métallique sont logées, lesdits tubes inférieurs étant positionnés à l'arrière de la bouche. Cet appareil présente toutefois plusieurs inconvénients. Premièrement, les tubes inférieurs étant positionnés dans la partie vestibulaire de la bouche gênent fortement le patient. Par ailleurs, le fait que les organes de fixation du maxillaire inférieur soient des tubes orientés vers l'avant de la bouche limite considérablement les possibilités d'ouverture de la bouche. Cet appareil ne permet pas non plus des déplacements latéraux mutuels des gouttières dentaires. De plus, le verrouillage des deux extrémités du fil soumet celui-ci à des efforts de torsion quasi-permanents qui peuvent aboutir à l'usage à de dangereuses ruptures dudit fil métallique dans les zones vestibulaires de la bouche et au désengagement intempestif des gouttières dentaires. Finalement, cet appareil ne permet pas d'adapter l'écart entre la gouttière inférieure et la gouttière supérieure de manière sûre et confortable pour le patient.

### Divulgation de l'invention

Le but de la présente invention est donc de proposer un appareil dentaire qui ne présente pas les inconvénients de l'art antérieur mentionné ci-dessus.

Dans ce but, l'appareil selon l'invention est caractérisé en ce qu'il comprend une gouttière supérieure et une gouttière inférieure destinées à chausser, respectivement, les dents de la mâchoire supérieure et de la mâchoire inférieure, dans lequel lesdites gouttières sont reliées entre elles au moyen d'un fil ressort possédant une forme sensiblement en U, ledit fil ressort étant disposé en position occlusale entre lesdites gouttières sans dépasser dans les zones vestibulaires de la bouche du patient, dans lequel chacune des extrémités du fil ressort est montée de manière pivotante sur une première desdites gouttières inférieure ou supérieure autour d'un axe de pivotement parallèle au plan occlusal de contact des dents des mâchoires inférieure et supérieure, dans lequel le fil ressort est relié de manière pivotante et amovible à une seconde desdites gouttières inférieure ou supérieure au moyen d'un crochet fixé de manière amovible ou non amovible sur ladite seconde gouttière, ledit crochet étant positionné de telle sorte qu'il produise un décalage entre les première et seconde gouttières dans au moins une direction perpendiculaire à l'axe de pivotement du fil ressort par rapport à la seconde gouttière.

D'autres variantes de l'invention sont définies dans les revendications dépendantes 2 à 20.

Ainsi configuré, l'appareil de l'invention n'encombre pas la partie vestibulaire de la bouche. En outre, il permet une certaine ouverture de la bouche du patient tout en limitant les efforts exercés sur le fil ressort reliant les gouttières inférieure et supérieure. Finalement, il permet de faire varier la position relative entre la gouttière inférieure et supérieure en fonction de la morphologie dentaire du patient.

### Brève description des dessins

On décrit ci-après, à simple titre d'exemple non limitatif, une forme d'exécution de l'appareil selon l'invention, ainsi que quelques variantes, en se référant au dessin annexé dans lequel :
Les figures 1 et 2 sont des vues, respectivement, de côté et en perspective d'une première forme d'exécution de l'appareil selon l'invention dans sa position normale d'utilisation,
La figure 3 est une vue en perspective de l'ensemble gouttière inférieure et fil ressort de l'appareil représenté sur les figures 1 et 2,
La figure 4 est une vue de dessus du fil ressort de l'appareil représenté sur les figures 1 et 2,
La figure 5 est une vue en perspective d'une deuxième forme d'exécution de l'appareil selon l'invention dans sa position normale d'utilisation,
La figure 6 est une vue en perspective de la gouttière inférieure de l'appareil représenté sur la figure 5.
Les figures 7a à 7e sont des vues agrandies du crochet utilisé dans l'appareil représenté sur la figure 5 au fur et à mesure de sa mise en place sur un élément de fixation correspondant de la gouttière inférieure.

### Description détaillée de modes d'exécution de l'invention

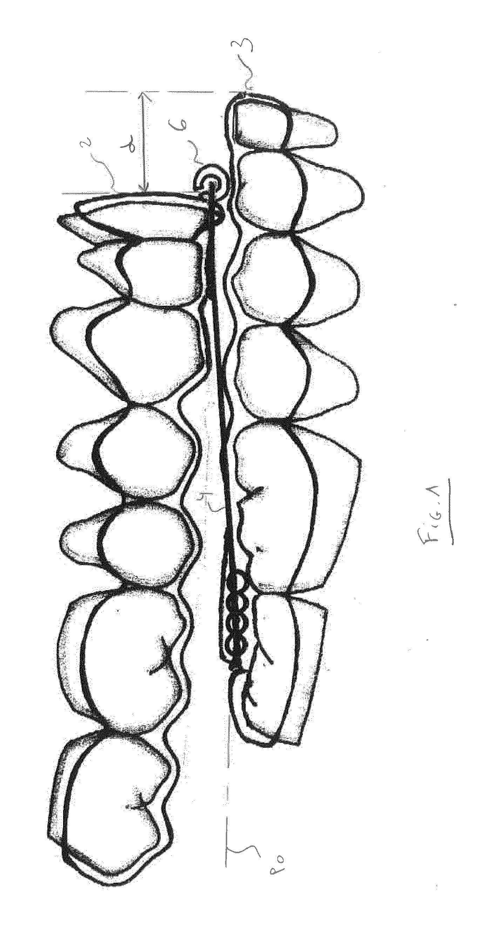

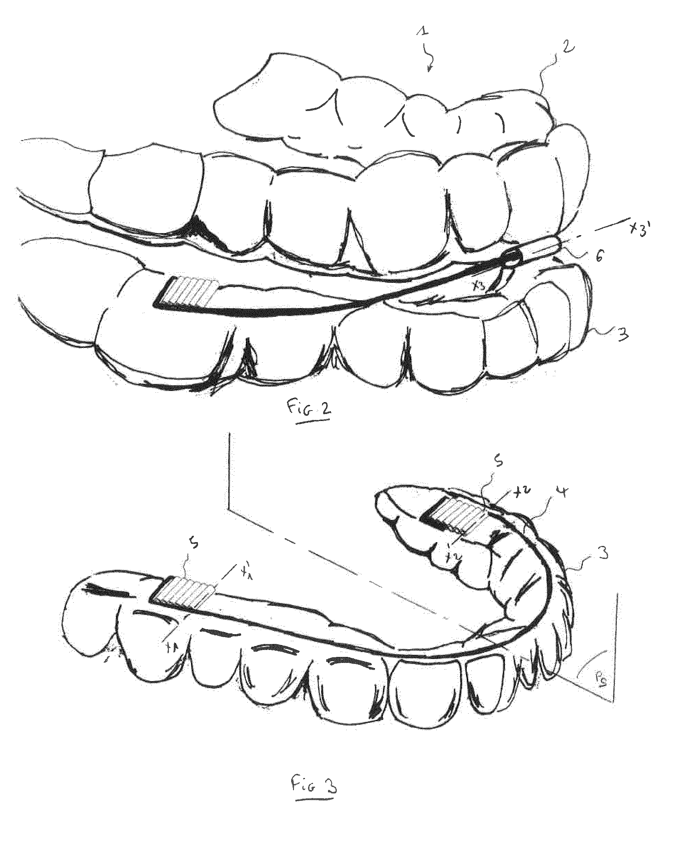

En se référant aux figures 1 et 2, on peut voir que l'appareil dentaire 1 mis en place dans la bouche d'un patient comprend une gouttière supérieure 2 et une gouttière inférieure 3 reliées au moyen d'un fil ressort 4. Chacune des gouttières 2, 3 reproduit en creux la forme des dentures des maxillaires supérieur et inférieur respectivement. Les gouttières 2, 3 sont réalisées par thermoformage suivant les caractéristiques morphologiques des mâchoires du patient en vue de s'adapter parfaitement à celles-ci. Les matériaux constitutifs des gouttières 2, 3 sont de préférence en résine acrylique synthétique ou sont constitués de plaques thermoformées biocompatibles.

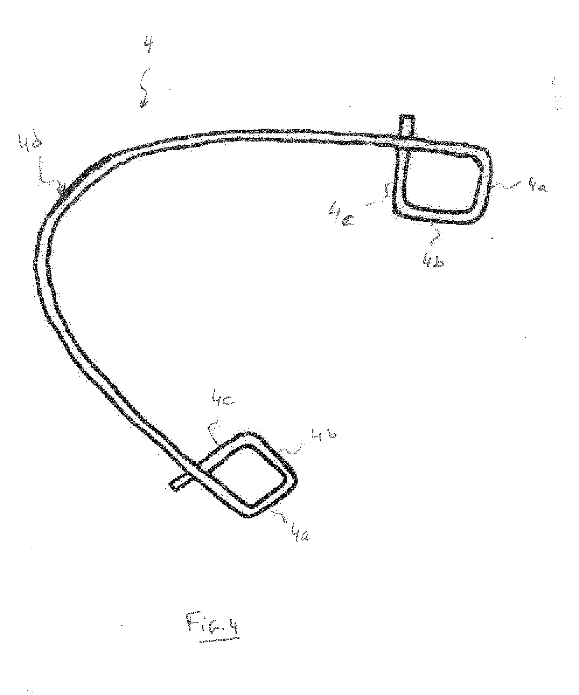

Tel que représenté sur les figures 3 et 4, le fil ressort 4 possède une forme sensiblement en U et est disposé en position occlusale entre les gouttières 2, 3 sans dépasser dans la partie vestibulaire de la bouche du patient. Ce fil ressort 4 pourra avantageusement être formé d'un métal possédant une bonne capacité de déformation élastique, en particulier d'un alliage nickel-titane. Ce fil ressort 4 pourra ainsi subir une torsion de telle sorte que ses extrémités s'écartent ou se rapprochent de la position d'équilibre représentée sur la figure 4, permettant ainsi la fixation amovible dudit fil ressort 4 sur la gouttière inférieure 3. Cette fixation pourra s'effectuer de plusieurs manières différentes. Dans la version représentée, cette fixation est réalisée au moyen d'une série de logements tubulaires 5 formés à l'intérieur de la gouttière inférieure 3, lesdits logements tubulaires 5 étant aptes à recevoir un segment d'extrémité 4c de chacune des branches du fil ressort 4. Il sera ainsi avantageux d'utiliser des logements tubulaires 5 dont le diamètre interne sera légèrement supérieur au diamètre du fil ressort 4, lequel sera compris entre 0.5 et 1.5 mm. De tels logements tubulaires 5 pourront avantageusement être formés au moyen de tubes creux noyés à l'intérieur de la gouttière inférieure 3. De manière préférentielle, les logements tubulaires 5 seront positionnés au niveau des premières et deuxièmes molaires inférieures et de manière symétrique par rapport à un plan de symétrie Ps de la gouttière inférieure 3. Ils seront avantageusement perpendiculaires ou sensiblement perpendiculaires à ce plan de symétrie Ps. Ainsi positionnés, les logements tubulaires 5 définiront deux axes de pivotement X1-X1' et X2-X2' autour desquels le fil ressort 4 pourra pivoter dès lors que les segments d'extrémité 4c auront été introduits à l'intérieur d'un des logements tubulaires 5. Les axes de pivotement X1-X1' et X2-X2' seront avantageusement parallèles au plan occlusal Po de contact des dents des mâchoires inférieure et supérieure. En fonction du degré d'avancement ou protrusion désiré, le patient pourra ainsi choisir d'introduire les segments d'extrémité 4c dans un logement tubulaire 5 plus ou moins éloigné de la partie frontale de la gouttière inférieure 3, laquelle sera en contact avec les lèvres inférieures de la bouche au niveau des incisives inférieures. Comme expliqué en détail par la suite, ce choix permettra de faire varier le décalage entre la gouttière supérieure 2 et la gouttière inférieure 3 dans une direction parallèle au plan Ps. Il convient de relever dès à présent que le choix du logement tubulaire le plus adapté et la fixation du fil ressort à l'intérieur de ce logement tubulaire peut s'effectuer par le patient lui-même, sans avoir recours à un professionnel praticien et sans utiliser d'outil spécifique. Ceci constitue donc un avantage supplémentaire de l'appareil dentaire de la présente invention vis-à-vis des appareils dentaires de l'art antérieur.

Dans une autre configuration possible de l'invention (non représentée), les extrémités du fil ressort 4 pourront être solidaires de la gouttière inférieure 3, notamment en noyant les extrémités 4c à l'intérieur de la gouttière inférieure 3. Toutefois, du fait de l'élasticité du fil ressort 4, il sera toujours possible d'opérer un pivotement dudit fil ressort 4 autour des axes X1-X1' et X2-X2'.

Tel que représenté sur les figures 1 et 2, la partie médiane 4d du fil ressort 4, correspondant sensiblement à la partie du fil ressort 4 qui est traversée par le plan Ps, est destinée à venir se loger à l'intérieur d'un crochet 6 solidaire de la gouttière supérieure 2. Ledit crochet 6 sera avantageusement positionné au niveau des incisives centrales supérieures et, de préférence, en étant fixé dans le prolongement de la face inférieure de la gouttière supérieure 2 qui fait face à la face supérieure de la gouttière inférieure 3, le crochet 6 dépassant légèrement du bord frontal de la gouttière supérieure 2. Dans cette position, il génère un décalage d entre la gouttière supérieure 2 et la gouttière inférieure 3, du fait que la partie médiane 4d du fil ressort 4, qui est logée à l'intérieur du crochet 6, est positionnée en retrait par rapport au bord frontal de la gouttière inférieure 3. Ce décalage d sera d'autant plus important que les segments d'extrémité 4c du fil ressort seront introduits dans les logements tubulaires 5 les plus éloignés dudit bord frontal. Comme représenté sur la figure 2, le crochet 6 pourra notamment posséder une partie hémi-tubulaire centrée autour d'un axe X3-X3' perpendiculaire au plan de symétrie de la gouttière supérieure 2. Dans la variante représentée, ce plan de symétrie est confondu avec le plan de symétrie Ps de la gouttière inférieure 3. Ainsi configuré, il sera possible de faire pivoter la gouttière supérieure 2 autour de l'axe X3-X3', tout en permettant un mouvement coulissant du crochet 6 le long du fil ressort 4. Ces possibilités supplémentaires de mouvement relatif entre les gouttières supérieure et inférieure amélioreront sensiblement le confort d'utilisation pour le patient.

Par ailleurs, afin d'éviter que le patient ne déconnecte accidentellement les gouttières supérieure et inférieure lors de son sommeil, notamment en ouvrant de manière trop importante la bouche, le fil ressort 4 sera avantageusement configuré pour limiter l'amplitude du pivotement relatif entre la gouttière supérieure 2 et la gouttière inférieure 3. A cet effet, et tel que représenté sur la figure 4, chacune des extrémités du fil ressort 4 possédera au moins trois segments successifs, à savoir :
- un premier segment 4a parallèle à l'axe de pivotement X1-X1' ou X2-X2',
- un deuxième segment 4b contigu et perpendiculaire au premier segment 4a,
- un troisième segment 4c contigu et perpendiculaire au deuxième segment 4b,
lesdits premier, deuxième et troisième segments 4a, 4b et 4c étant alignés dans un plan qui est parallèle au plan occlusal Po ou légèrement incliné par rapport au plan occlusal Po, l'angle d'inclinaison étant inférieur à 5°. Comme mentionné précédemment, le troisième segment 4c de chacune des extrémités du fil ressort 4 est destiné à être introduit à l'intérieur d'un des logements tubulaires 5 qui sont formés à l'intérieur de la gouttière inférieure 3, permettant ainsi le pivotement du fil ressort 4 autour des axes X1-X1' et X2-X2'. Toutefois, au fur et à mesure du pivotement du fil ressort 4 autour des axes X1-X1' et X2-X2', le deuxième segment 4b se rapproche de la gouttière inférieure 3 jusqu'à venir buter contre la surface supérieure de ladite gouttière inférieure 3 dans une position limite du fil ressort 4 correspondant à l'amplitude maximale d'ouverture de la bouche du patient. Ainsi configuré, le fil ressort 4 autorise une ouverture suffisante de la bouche tout en limitant les risques d'une déconnexion des gouttières inférieure et supérieure.

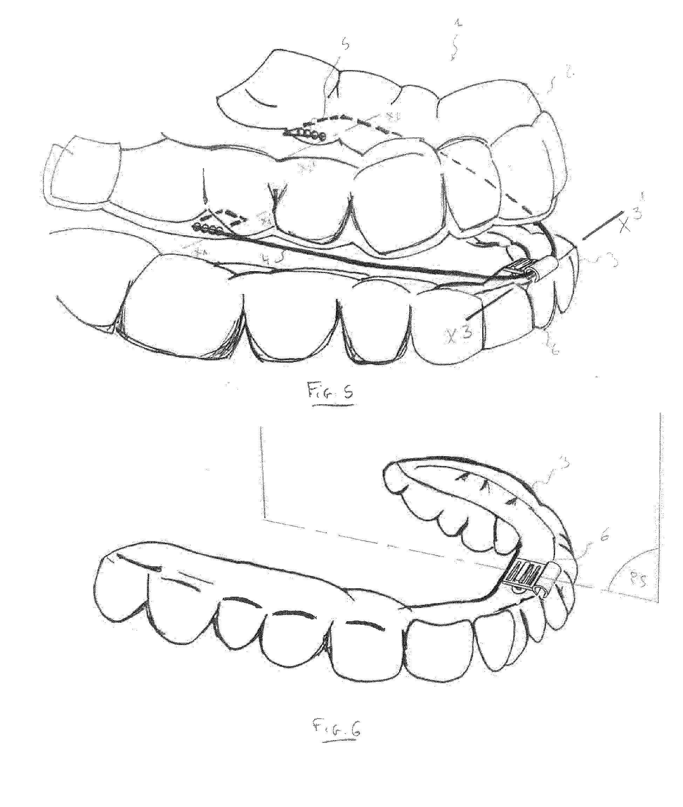

Les figures 5 et 6 représentent une variante d'exécution possible pour l'appareil dentaire de la présente invention.

Dans cette variante, les logements tubulaires 5 sont formés à l'intérieur de la gouttière supérieure 2 et le crochet 6 est fixé de manière amovible sur la gouttière inférieure 3. Ainsi, dans sa position d'utilisation, le fil ressort 4 vient buter contre les parois internes de la partie hémi-tubulaire du crochet 6. Contrairement à la variante précédente, dans laquelle le fil ressort 4 était soumis à un effort de traction, dans la présente variante, le fil ressort 4 est soumis à un effort de compression. Du fait de cet effort de compression, le fil ressort 4 aura tendance à se déformer de telle sorte que ses branches s'écartent en direction des zones vestibulaires de la bouche. Cette variante peut donc, dans certains cas, s'avérer moins confortable à l'usage que la première variante. De manière avantageuse, le crochet 6 est fixé sur la gouttière inférieure 3 de telle sorte que sa position selon une direction parallèle au plan de symétrie Ps, tel que défini précédemment, soit variable. Ainsi, l'utilisateur pourra avancer ou reculer le crochet 6, offrant ainsi une possibilité supplémentaire pour le patient de faire varier les positions relatives des gouttières supérieure et inférieure 2 et 3. Il est évident qu'une telle possibilité supplémentaire pourra également être envisagée dans la première variante d'exécution représentée sur les figures 1 et 2, ainsi que dans une autre variante d'exécution (non représentée), dans laquelle les extrémités du fil ressort 4 sont solidaires de la gouttière supérieure 2, dans le cas où le crochet 6 est fixé de manière amovible à la gouttière inférieure 3, ou de la gouttière inférieure 3, dans le cas où le crochet 6 est fixé de manière amovible à la gouttière supérieure 2.

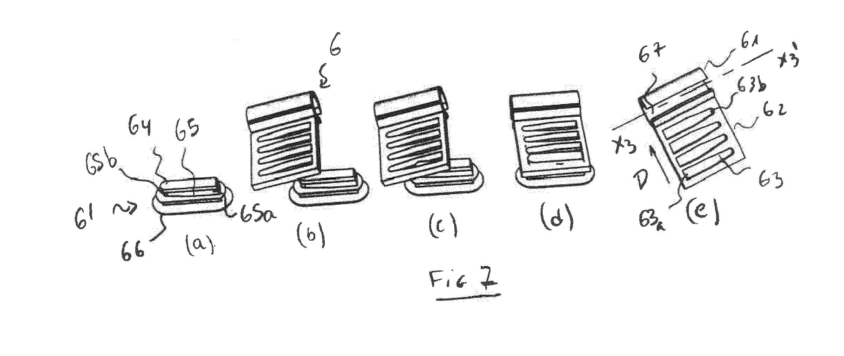

La fixation amovible du crochet 6 sur la gouttière supérieure 2 ou inférieure 3 pourra s'effectuer de plusieurs manières différentes. Dans la variante représentée sur les figures 5 et 6, et tel qu'illustré en détail sur les figures 7a à 7e, le crochet 6 est formé d'une partie hémi-tubulaire 61 définissant une ouverture axiale 67 et solidaire d'une plaque 62 sensiblement rectangulaire, ladite plaque 62 étant percée de plusieurs ouvertures 63 régulièrement espacées dans une direction longitudinale D perpendiculaire à l'axe X3-X3' défini par la partie 61. Comme représenté sur la figure 7e, les ouvertures 63 ont sensiblement la même forme, ladite forme étant définie par une extrémité large 63a et par une extrémité étroite 63b. Les ouvertures 63 sont en particulier configurées pour recevoir une partie médiane 65 d'un élément de fixation 6' qui est solidaire d'une des gouttières 2, 3 de l'appareil dentaire de l'invention. De manière avantageuse, la partie médiane 65 possède une forme correspondant sensiblement à la forme des ouvertures 63, de sorte qu'elle est définie par une extrémité large 65a et par une extrémité étroite 65b. L'élément 6' comprend également une partie inférieure 66, destinée à être noyée à l'intérieur de ladite gouttière, et une partie supérieure 64, destinée à coiffer la partie médiane 65 de telle sorte qu'elle déborde de chaque côté de son extrémité étroite 65b. Ainsi, comme représenté sur les figures 7b, 7c et 7d, le crochet 6 sera fixé sur l'élément de fixation 6' en alignant la partie supérieure 64 de l'élément de fixation 6' avec l'une des ouvertures 63 (voir figure 7b), en faisant partiellement passer ladite partie supérieure 64 à travers ladite ouverture 63 au niveau de l'extrémité large 63a (voir figure 7c), en déplaçant la plaque 62 du crochet 6 parallèlement à l'axe X3-X3' jusqu'à ce que l'extrémité étroite 65b de la partie médiane 65 vienne en butée contre l'extrémité étroite 63b de l'ouverture 63 (voir figure 7d), et finalement en faisant basculer la plaque 62 jusqu'à ce que la partie supérieure 64 ait entièrement passé à travers l'ouverture 63 et soit positionnée au-dessus de la plaque 62, dans le cas où l'ouverture 67 est dirigée vers le bas, comme illustré sur la figures 6, ou soit positionnée au-dessous de la plaque 62, dans le cas où l'ouverture 67 est dirigée vers le haut.

Il va de soi que l'invention n'est pas limitée aux formes de réalisation décrites ci-dessus à titre d'exemple mais qu'elle en embrasse, au contraire, toutes les variantes de réalisation.

## Revendications

1. Appareil dentaire (1) pour le réglage du positionnement de la mâchoire inférieure d'un patient par rapport à sa mâchoire supérieure, ledit appareil comprenant une gouttière supérieure (2) et une gouttière inférieure (3) destinées à chausser, respectivement, les dents de la mâchoire supérieure et de la mâchoire inférieure, dans lequel lesdites gouttières (2, 3) sont reliées entre elles au moyen d'un fil ressort (4) possédant une forme sensiblement en U, ledit fil ressort (4) étant disposé en position occlusale entre lesdites gouttières (2, 3) sans dépasser dans les zones vestibulaires de la bouche du patient, dans lequel chacune des extrémités (4c) du fil ressort (4) est montée de manière pivotante sur une première desdites gouttières inférieure (3) ou supérieure (2) autour d'un axe de pivotement (X1 X1' ; X2X2') parallèle au plan occlusal (Po) de contact des dents des mâchoires inférieure et supérieure, dans lequel le fil ressort (4) est relié de manière pivotante et amovible à une seconde desdites gouttières inférieure (3) ou supérieure (2) au moyen d'un crochet (6) fixé de manière amovible ou non amovible sur ladite seconde gouttière, ledit crochet (6) étant positionné de telle sorte qu'il produise un décalage (d) entre les première et seconde gouttières (2, 3) dans au moins une direction perpendiculaire à l'axe de pivotement (X3X3') du fil ressort (4) par rapport à la seconde gouttière.

2. Appareil (1) selon la revendication 1, **caractérisé en ce que** chacune des extrémités du fil ressort (4) est montée de manière amovible sur ladite première gouttière.

3. Appareil (1) selon la revendication 2, **caractérisé en ce que** ladite première gouttière possède plusieurs paires de logements tubulaires (5), chacune desdites paires étant apte à recevoir les extrémités (4c) du fil ressort (4), lesdites paires permettant ainsi de faire varier la position des axes de pivotement (X1X1' ; X2X2') de chacune desdites extrémités et, de ce fait, de faire varier le décalage entre la première gouttière et la seconde gouttière.

4. Appareil (1) selon l'une quelconque des revendications 1 à 3, **caractérisé en ce que** chacune des extrémités du fil ressort est conformée de manière à définir une butée (4b) limitant le pivotement dudit fil ressort par rapport à la première gouttière.

5. Appareil (1) selon la revendication 4, **caractérisé en ce que** chacune des extrémités du fil ressort possède au moins trois segments successifs :
- un premier segment (4a) parallèle à l'axe de pivotement d'une desdites extrémités,
- un deuxième segment (4b) contigu et perpendiculaire au premier segment,
- un troisième segment (4c) contigu et perpendiculaire au deuxième segment,
lesdits premier, deuxième et troisième segments (4a, 4b, 4c) étant alignés dans un plan qui est parallèle au plan occlusal (Po) de contact des dents des mâchoires inférieure et supérieure ou légèrement incliné par rapport audit plan occlusal (Po), l'angle d'inclinaison étant inférieur à 5°, **en ce qu'**au moins une paire de logements tubulaires (5) formés à l'intérieur de ladite première gouttière est apte à recevoir les troisièmes segments (4c) desdites extrémités, lesdits logements tubulaires (5) définissant ainsi les axes de pivotement (X1X1' ; X2X2') desdites extrémités, et **en ce que** le fil ressort (4) est apte à pivoter autour desdits axes de pivotement (X1 X1' ; X2X2') jusqu'à ce que les deuxièmes segments (4b) desdites extrémités viennent buter contre ladite première gouttière.

6. Appareil (1) selon la revendication 5, **caractérisé en ce que** la première gouttière possède plusieurs paires de logements tubulaires (5), chacune desdites paires étant apte à recevoir les troisièmes segments (4c) des extrémités du fil ressort (4), lesdites paires permettant ainsi de faire varier la position des axes de pivotement (X1X1' ; X2X2') de chacune desdites extrémités et, de ce fait, de faire varier le décalage entre la première gouttière et la seconde gouttière.

7. Appareil (1) selon l'une quelconque des revendications 3, 5 et 6, **caractérisé en ce que** les logements tubulaires (5) sont formés par des tubes creux noyés à l'intérieur de la première gouttière.

8. Appareil (1) selon l'une quelconque des revendications précédentes, **caractérisé en ce que** la première gouttière et/ou la seconde gouttière possède un plan de symétrie (Ps).

9. Appareil (1) selon la revendication 8, **caractérisé en ce que** les axes de pivotement (X1X1' ; X2X2') des extrémités du fil ressort (4) sont perpendiculaires ou sensiblement perpendiculaires au plan de symétrie (Ps) de la première gouttière.

10. Appareil (1) selon l'une quelconque des revendications 8 et 9, **caractérisé en ce que** le crochet (6) possède une partie hémi-tubulaire (61) centrée autour d'un axe (X3X3') perpendiculaire au plan de symétrie (Ps) de la seconde gouttière, ladite partie hémi-tubulaire définissant une fente (67) et étant apte à loger au moins partiellement le fil ressort (4).

11. Appareil (1) selon la revendication 1, **caractérisé en ce que** chacune des extrémités du fil ressort (4) est solidaire de la première gouttière.

12. Appareil (1) selon l'une quelconque des revendications 1 à 11, **caractérisé en ce que** le crochet (6) est solidaire de la seconde gouttière.

13. Appareil (1) selon l'une quelconque des revendications 1 à 11, **caractérisé en ce que** le crochet (6) est fixé de manière amovible sur la seconde gouttière.

14. Appareil (1) selon la revendication 13, **caractérisé en ce que** le crochet (6) possède une première partie hémi-tubulaire (61) et une seconde partie (62) de forme plane, ladite partie hémi-tubulaire étant apte à loger au moins partiellement le fil ressort (4) et ladite seconde partie (62) étant apte à loger au moins partiellement un élément de fixation (6') de la seconde gouttière.

15. Appareil (1) selon la revendication 14, **caractérisé en ce que** ledit élément de fixation (6') possède une partie médiane (65) apte à être reçue à l'intérieur d'une ouverture (63) formée à l'intérieur de la partie plane (62) du crochet (6), ladite partie médiane (65) et ladite ouverture (63) possédant sensiblement la même forme.

16. Appareil (1) selon la revendication 15, **caractérisé en ce que** la partie plane (62) du crochet (6) est percée d'une pluralité d'ouvertures (63) de forme identique, régulièrement espacées le long d'une direction (D) perpendiculaire à la direction axiale (X3X3') définie par la partie hémi-tubulaire (61) du crochet (6).
